# EUROPEAN PATENT APPLICATION

(11) **EP 2 156 862 A1**
(43) Date of publication of application: **24.02.2010**
(21) Application number: 09165729.6
(22) Date of filing: 22.03.2007
(51) Int. Cl.: A61P 25/18, A61P 25/22, A61P 25/24, A61K 31/585

(54) **Use of digitalis-like compounds in the treatment of affective disorders**

(30) Priority: 23.03.2006 US 784782 P
(62) Divisional of application: 07736128.5
(71) Applicant: Yissum Research Development Company of The Hebrew University of Jerusalem, 91390 Jerusalem (IL)
(72) Inventor: Lichtstein, David, 90855, Moshav Shoeva (IL); Rosen, Haim, 96956, Jerusalem (IL)
(74) Representative: Fichter, Robert Arno

(57) **Abstract**

Use of digitalis-like compounds such as 19-norbufalin derivatives in the treatment of affective disorders such as anxiety, depression and bipolar disorders is disclosed.

## Description

### FIELD AND BACKGROUND OF THE INVENTION

The present invention relates to synthetic digitalis analogs and, more particularly, to the use of synthetic digitalis analogs in the treatment of affective disorders such as bipolar affective disorders, depression and anxiety.

Cardenolides and bufadienolides are steroid compounds originally isolated from plants and toad skin respectively, and are collectively termed herein and in the art "digitalis-like compounds" (DLCs).

DLCs are well-known as inhibitors of the plasma membrane Na⁺-K⁺-ATPase. The Na⁺-K⁺-ATPase (E.C.3.6.1.3) is an integral plasma membrane protein which is responsible for maintaining sodium and potassium ions gradient across cell membranes in all eukaryotic cells. This enzyme has a high-affinity receptor for digitalis steroids. Endogenous ligands for these receptors have therefore been postulated for regulating the Na⁺-K⁺-pump activity.

Indeed, digitalis-like compounds have been shown to be present in diverse mammalian and amphibian tissues.

More recently, DLCs have been shown to be present in human tissue. Ouabain was identified in human plasma and adrenal tissue [Hamlyn et al. Proc. Natl. Acad. Sci. USA 1991, 88:6259-6263; Sich et al. Hypertension 1996, 27:1073-1078], digoxin was found in urine [Goto et al. Biochem. Biophys. Res. Commun. 1990, 173:1093-1101], 19-norbufalin and a derivative thereof were identified in cataractous lenses [Lichtstein et al. Eur. J Biochem. 1993, 216:261-268], and dihydropyrone-substituted bufadienolide was identified in placenta [Hilton et al. Lancet 1996, 348:303-305].

DLCs have therefore been considered as mammalian steroid hormones, synthesized and released by the adrenal glands, which regulate functions such as heart rate and blood pressure by inhibiting Na⁺-K⁺-ATPase, although they may have other molecular targets as well [Schoner Eur. J. Biochem. 2002, 269:2440-2448].

Naturally occurring and synthetic digitalis-like compounds are therefore suggested to be used as therapeutic agent in the treatment of various pathological conditions in which involvement of endogenous digitalis-like compounds is implied. Such compounds may therefore be used as cardiotonic agents, increasing the intensity of heart muscle contractions, as vasoactive agents, elevating blood pressure and as natriuretic/diuretic agents, increasing the excretion of sodium into the urine and thus increasing urine volume.

Inhibition of the Na⁺-K⁺-ATPase is commonly performed in biological research, typically using ouabain, and in cardiac medicine, typically using digoxin, a digitalis glycoside.

Digitalis glycosides share the property of being toxic immediately above their therapeutic range. Toxic effects of these drugs include: arrhythmias, ECG effects such as increased blood pressure and heart rate, pulmonary congestion, delirium, fatigue, disturbance of color vision, anorexia, nausea, and vomiting. These drugs are cardiotoxic and neurotoxic because of their effect on the sympathetic nervous system.

Digoxin is widely used as an inotropic drug to treat heart failure. Its major drawback, however, lies is the possible emergence of digoxin intoxication, due to high levels of digoxin in the plasma, which triggers heart arrhythmia and death. Digitalis like compounds devoid of such an intoxication effect have therefore been long sought for.

U.S. Patent No. 7,087,590, to Lichtstein et al., which is incorporated by reference as if fully set forth herein, discloses novel 19-norbufalin derivatives and processes of preparing same. According to the teachings of this patent, the norbufalin derivatives taught therein are very similar in structure to the known digitalis and bufodienolides and were found to similarly act as inhibitors of Na⁺-K⁺-ATPase activity. Thus, these compounds were shown to have, like the known cardienolides, effects on cardiac and smooth muscle contractility and kidney and neuronal function and hence were suggested for use as drugs affecting the cardiovascular and other systems involving the Na⁺-K⁺-ATPase.

Thus, these compounds were suggested as being agents for treating cardiac and renal malfunctions involving Na⁺-K⁺-ATPase, such as arrhythmia and cardiac failure, induction of natriuresis and diuresis, and constriction of smooth muscle in arterioles, causing elevation of blood pressure. In addition, these compounds were suggested to be used as neuromodulators, affecting the central nervous system. These compounds were further found to have an effect on cell differentiation and have therefore been suggested to be used as agents for the treatment of various proliferative cell and malignant diseases.

Further according to the teachings of U.S. Patent No. 7,087,590, the 19-norbufalin derivatives described therein were found to be considerably less toxic than the abundantly used digoxin. Moreover, some of these compounds were shown to exhibit an antagonistic activity towards digoxin, and thus to act as agents for treating digoxin intoxication.

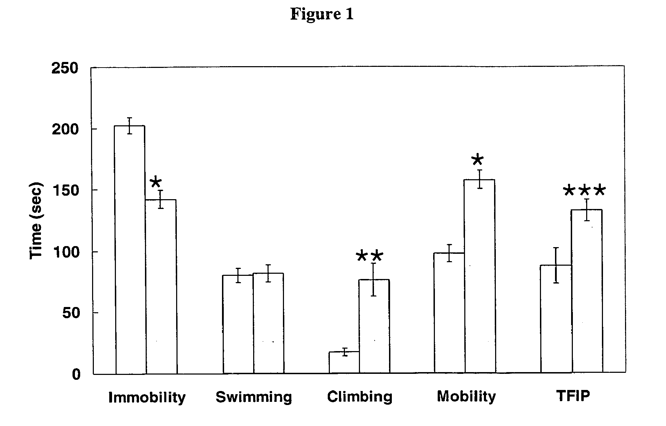

More specifically, it has been found that the novel 19-norbufalin derivatives disclosed in U.S. Patent No. 7,087,590 can be obtained as both the α and β isomers thereof (see, for example, Figures 1 and 3 therein). Natural DLCs have the β configuration. Indeed, it was found that β isomers exhibit a similar activity to that of digoxin, but with less toxicity, whereby the α isomers, while being devoid of digoxin-like activity and being also non-toxic, prevent the effect of digoxin and hence presumably antagonize the activity of digoxin.

While, as discussed hereinabove, digoxin toxicity is a significant problem due to the widespread therapeutic use of digoxin, the 19-norbufalin derivatives disclosed in U.S. Patent No. 7,087,590 are proposed therein as a treatment for digoxin intoxication.

U.S. Patent Nos. 5,5567,679 and 5, 591,734 (see also EP Patent No. 583578), as well as Ferrari et al. [JPET, 285:83-94, 1998], disclose 17-(3-furyl/pyradinizyl) 5β, 14β-androstane derivatives, which were found to act as antagonists of the effect of ouabain. These derivatives are taught in these documents as agents for treating cardiovascular disorders such as heart failure and hypertension.

Further according to the teachings of U.S. Patent No. 7,087,590, and as mentioned hereinabove, based on the marked involvement of Na⁺-K⁺-ATPase in the central nervous system, the compounds taught therein were suggested to act as neuromodulators, and hence as possible agents for the treatment of CNS disorders. However, a clear indication for an effect of DLCs on certain CNS has not been described nor demonstrated in this publication.

The neurological effects of DLCs have been studied to some extent. Thus, it has been found that therapeutic use of digitalis may cause mania or depression as side effects [Keller and Frishman Cardiol. Rev. 2003, 11:73-93; Schleifer et al. Am. Heart J. 1991, 121:1397-1402]. In addition, certain natural bufadienolides are abused as addictive substances.

Further, it has been reported that ouabain induces both manic and depressive activity in rats [El-Mallakh et al. Prog. Neuropsychopharmacol. Biol. Psychiatry 1995, 19:955-962; Li et al. Mol. Chem. Neuropathol. 1997, 31:65-72] and in cultured neurons [El-Mallakh et al. J. Psychiatr. Res. 2000, 34:115-120]. It was found that in both these *in vivo* and *in vitro* models, these effects are blocked by lithium, leading to the hypothesis that these effects are related to the manic and depressive phases of bipolar disorder [Li et al. Mol. Chem. Neuropathol. 1997, 31:65-72; El-Mallakh et al. J. Psychiatr. Res. 2000, 34:115-120].

Furthermore, bipolar patients have been found to have reduced Na⁺-K⁺-ATPase activity during manic and depressive stages [Looney and El-Mallakh Depress. Anxiety 1997, 5:53-65], and bipolar disorder has been linked to mutated Na⁺-K⁺-ATPase [Mynett-Johnson et al. Biol. Psychiatry 1998, 44:47-51], consistent with the effects of ouabain and other DLCs.

However, the connection between DLCs and bipolar disorder is complex. DLC levels have been found to be increased in the brains of bipolar patients [Goldstein et al. Biol. Psychiatry 2006, 60:491-499], but decreased in their plasma [Grider et al. J. Affect. Disord. 1999, 54:261-267]. The increased DLC levels in brain are accompanied by decreased affinity of Na⁺-K⁺-ATPase to DLCs [Goldstein et al. 2006, *supra*]. It has therefore been thought that the decreased affinity is a mechanism of compensating for high DLC levels, or otherwise that high DLC levels are a mechanism for compensating for decreased affinity. In addition, it has been found that both ouabain and anti-ouabain antibodies reduced depression in the same rat model, and these findings were thought to be attributed to the fact that ouabain stimulates Na⁺-K⁺-ATPase at low concentrations, whereby other DLCs may not, or to the possible inhibition, by anti-ouabain antibodies, of DLCs with different effects than ouabain [Goldstein et al. 2006, *supra*]. Differences between the effects of various DLCs, differences in the DLC levels in various parts of the body, and potential compensatory mechanisms in the body make it difficult to combine the results of these various studies to produce a clear view of the relationship of DLCs and bipolar disorder and related affective disorders.

Thus, to date, current findings fail to provide a clear indication regarding the effect of DLCs on CNS disorders in general and affective disorders in particular, but suggest that DLCs are involved in inducing bipolar disorders.

Antidepressants are used to treat depression resulting from both clinical depression and bipolar disorders, as well as other affective disorders such as anxiety disorders and eating disorders. Almost all antidepressants currently used may be classified as 3 families, selective serotonin reuptake inhibitors (SSRI), tricyclics, and monoamine oxidase inhibitors (MAOI). All of these families cause significant side effects. Even SSRIs, which are popular due to their relatively few side effects, commonly cause adverse side effects such as sexual dysfunction, and are suspected of increasing suicidal tendencies in some patients. Moreover, some studies reported that antidepressants produce only slightly better results than placebos [Moncrieff and Kirsch, BMJ 2005, 331:155-157].

Electroconvulsive therapy is a relatively effective treatment for depression, although its mechanism remains unknown. However, this treatment is used only reluctantly due to the adverse side effect of memory loss and the poor public image associated with this treatment.

Bipolar disorders are generally treated with mood stabilizers such as lithium, anticonvulsants and atypical antipsychotics. Mood stabilizers are typically more effective at preventing mania than depression, and as a result, antidepressants are often prescribed along with mood stabilizers. However, the additional use of antidepressants increases the number of potential side effects, may induce mania, and may worsen the long-term prognosis of the disease [Ghaemi and Goodwin, Am. J. Psychiatry 2005, 162:1545-1546]. In addition, mood stabilizers have significant side effects. The therapeutic dose of lithium is close to the toxic dose, requiring regular blood testing. Anticonvulsants are thought to have less severe side effects than lithium, but may cause sedation, weight gain and electrolyte disturbances, and are thought to be less effective at preventing depression.

There is thus a widely recognized need for, and it would be highly advantageous to have, novel agents for treating affective disorders, devoid of the above limitations.

### SUMMARY OF THE INVENTION

The present inventors have now surprisingly found that DLCs such as, for example, 19-norbufalin derivatives, can be beneficially used in the treatment of affective disorders such as bipolar disorders, depression and anxiety.

Thus, according to one aspect of the present invention there is provided a method of treating an affective disorder in a subject in need thereof, the method comprising administering to the subject a therapeutically effective amount of a digitalis-like compound.

According to another aspect of the present invention there is provided a use of a digitalis-like compound in the manufacture of a medicament for treating an affective disorder.

According to yet another aspect of the present invention there is provided a pharmaceutical composition, packaged in a packaging material, and identified in print, in or on the packaging material, for use in the treatment of an affective disorder, the composition comprising, as an active ingredient, a digitalis-like compound and a pharmaceutically acceptable carrier.

According to further features in preferred embodiments of the invention described below, the affective disorder is selected from the group consisting of a bipolar disorder, a body dysmorphic disorder, a bulimia nervosa, an eating disorder, cataplexy, cyclothymia, dysthymia, a general anxiety disorder, a major depressive disorder, an obsessive compulsive disorder, a panic disorder, a post-traumatic stress disorder, a premenstrual dysphoric disorder, and a social phobia.

According to still further features in the described preferred embodiments the digitalis-like compound has the general Formula I: 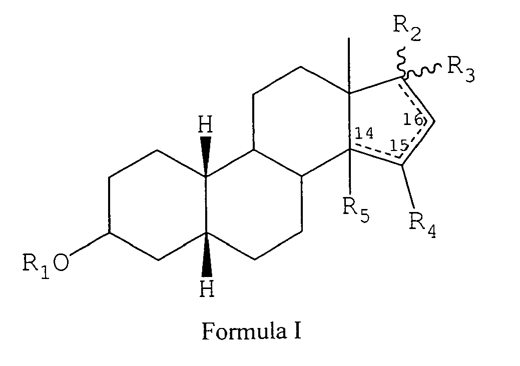 wherein:
the dashed line represents an optional double bond;
R₁ is hydrogen, alkyl, alkenyl, cycloalkyl or a hydroxy protecting group or is absent (forming an oxo group);
R₂ is hydrogen, hydroxy, alkoxy, aminoalkyl or absent;
R₃ is selected from the group consisting of furyl, dihydrofuryl, tetrahydrofuryl, pyranyl, dihydropyranyl, tetrahydropyranyl, pyridanyzil and lactone;
R₄ is hydrogen or hydroxy, or, alternatively, forms a 3-membered ring with R₅; and
R₅ is hydrogen, hydroxy or absent, or, alternatively, forms a 3-membered ring with R₅.

According to still further features in the described preferred embodiments the compound is in a form of an alpha isomer or a beta isomer thereof.

According to still further features in the described preferred embodiments, in general Formula I, R₁ is hydrogen or a hydroxy protecting group; R₂ is hydrogen, hydroxy or absent; R₃ is selected from the group consisting of: 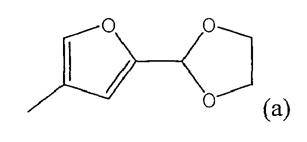 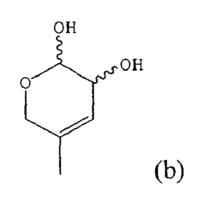 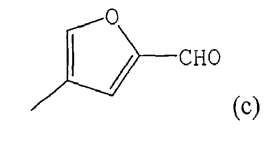 and

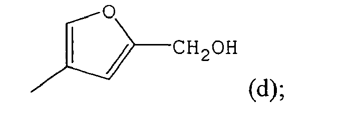

R₄ is hydrogen or hydroxy; R₅ is hydrogen, hydroxy or absent; and the dashed line represents an optional double bond.

According to still further features in the described preferred embodiments the compound is in a form of an alpha-isomer thereof.

According to still further features in the described preferred embodiments the hydroxy protecting group is selected from the group consisting of benzyl, amino acid, peptide, and mono- and di-saccharide.

According to still further features in the described preferred embodiments R₁ is benzyl.

According to still further features in the described preferred embodiments R₂ is OH.

According to still further features in the described preferred embodiments R₃ is (a).

According to still further features in the described preferred embodiments R₄ and R₅ are each hydrogen, the compound having a double bound between the carbons at the 15 and 16 positions.

According to still further features in the described preferred embodiments R₄ is hydrogen and R₅ is absent, the compound having a double bond between carbon atoms at the 14 and 15 positions.

According to still further features in the described preferred embodiments R₄ is hydroxy, and R₅ is hydrogen.

According to still further features in the described preferred embodiments R₁ is hydrogen.

According to still further features in the described preferred embodiments R₂ is hydrogen.

According to still further features in the described preferred embodiments R₃ is (d).

According to still further features in the described preferred embodiments R₄ is hydroxy and R₅ is hydrogen.

According to still further features in the described preferred embodiments R₃ is (b).

According to still further features in the described preferred embodiments R₄ is hydrogen and R₅ is absent, the compound having a double bond between the carbon atoms at the 14 and 15 positions.

According to still further features in the described preferred embodiments R₃ is (c).

According to still further features in the described preferred embodiments R₄ is hydrogen and R₅ is absent, the compound having a double bond between the carbon atoms at the 14 and 15 positions.

According to still further features in the described preferred embodiments R₃ is (c).

According to still further features in the described preferred embodiments R₄ is hydrogen and R₅ is hydroxy.

The present invention successfully addresses the shortcomings of the presently known configurations by providing methods and compositions for treating affective disorders which utilize non-toxic, highly efficacious digitalis-like compounds.

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below. In case of conflict, the patent specification, including definitions, will control. In addition, the materials, methods, and examples are illustrative only and not intended to be limiting.

As used herein, the singular form "a", "an" and "the" include plural references unless the context clearly dictates otherwise. For example, the term "a protein" or "at least one protein" may include a plurality of proteins, including mixtures thereof.

As used herein the term "about" refers to ± 10 %.

Throughout this disclosure, various aspects of this invention can be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

Whenever a numerical range is indicated herein, it is meant to include any cited numeral (fractional or integral) within the indicated range. The phrases "ranging/ranges between" a first indicate number and a second indicate number and "ranging/ranges from" a first indicate number "to" a second indicate number are used herein interchangeably and are meant to include the first and second indicated numbers and all the fractional and integral numerals therebetween.

As used herein throughout, the term "comprising" means that other steps and ingredients that do not affect the final result can be added. This term encompasses the terms "consisting of" and "consisting essentially of'.

The phrase "consisting essentially of" means that the composition or method may include additional ingredients and/or steps, but only if the additional ingredients and/or steps do not materially alter the basic and novel characteristics of the claimed composition or method.

The term "method" or "process" refers to manners, means, techniques and procedures for accomplishing a given task including, but not limited to, those manners, means, techniques and procedures either known to, or readily developed from known manners, means, techniques and procedures by practitioners of the chemical, pharmacological, biological, biochemical and medical arts.

### BRIEF DESCRIPTION OF THE DRAWINGS

The invention is herein described, by way of example only, with reference to the accompanying drawings. With specific reference now to the drawings in detail, it is stressed that the particulars shown are by way of example and for purposes of illustrative discussion of the preferred embodiments of the present invention only, and are presented in the cause of providing what is believed to be the most useful and readily understood description of the principles and conceptual aspects of the invention. In this regard, no attempt is made to show structural details of the invention in more detail than is necessary for a fundamental understanding of the invention, the description taken with the drawings making apparent to those skilled in the art how the several forms of the invention may be embodied in practice.

In the drawings:
FIG. 1 is a bar graph presenting the effect of intraperitoneal (i.p) injection of Compound 13-3 (gray bars), as compared to the vehicle only (open bars), on forced swimming behavioral test in rats. Results are expressed as Mean ± SEM (5 rats).

### DESCRIPTION OF THE PREFERRED EMBODIMENTS

The present invention is of a novel use of digitalis-like compounds (DLCs), and, specifically is of a use of digitalis-like compounds such as, for example, 19-norbufalin derivatives, in the treatment of affective disorders, such as bipolar disorder, clinical depression and anxiety.

The principles and operation of the present invention may be better understood with reference to the drawing and accompanying descriptions.

Before explaining at least one embodiment of the invention in detail, it is to be understood that the invention is not limited in its application to the details set forth in the following description or exemplified by the Examples. The invention is capable of other embodiments or of being practiced or carried out in various ways. Also, it is to be understood that the phraseology and terminology employed herein is for the purpose of description and should not be regarded as limiting.

As discussed hereinabove, currently used antidepressants are severely limited by their frequent induction of side effects and by their limited potency.

As further discussed hereinabove, DLCs have been shown to be associated with disorders such as bipolar disorders and depression, with contradictory findings regarding their involvement such disorders.

As further discussed hereinabove, recently, a novel family of synthetic DLCs, 19-norbufalin derivatives, has been disclosed in U.S. Patent No. 7,087,590. These 19-norbufalin derivatives were found to exhibit, *inter alia*, an activity that antagonizes the effect digoxin and hence were suggested for use as agents for treating or preventing digoxin intoxification. In addition, androstane derivatives that are capable of antagonizing the effect of endogenous digitalis like compounds such as ouabain are disclosed, for example, in U.S. Patent Nos. 5,5567,679 and 5,591,734.

In a search for novel agents for use in the treatment of affective disorders, which would be both potent and safe (non-toxic), and would further be devoid of adverse side effected, the present inventors have studied the anti-depressive effect of synthetic DLCs.

Indeed, as demonstrated in the Examples section that follows, while reducing the present invention to practice, digitalis-like compounds were found to have an anti-depressive effect in a well-recognized animal model.

Accordingly, according to one aspect of the present invention, there is provided a method of treating an affective disorder. The method is effected by administering to a subject in need thereof a therapeutically effective amount of a digitalis-like compound (DLC).

As used herein, the terms "treating", "treatment" and other grammatical diversions thereof include abrogating, substantially inhibiting, slowing or reversing the progression of a condition, substantially ameliorating clinical or aesthetical symptoms of a condition or substantially preventing the appearance of clinical or aesthetical symptoms of a condition.

As used herein, the phrase "affective disorder" collectively describes any psychological and/or psychotic disorder characterized by an undesirable excess of emotions such as, but not limited to, sadness, fear, anxiety and self-loathing, and encompasses any disorder that may be treated effectively by essentially the same treatments as the aforementioned disorders.

Non-limiting examples of affective disorders include bipolar disorders (e.g., manic depression), body dysmorphic disorders, bulimia nervosa and other eating disorders, cataplexy, cyclothymia, dysthymia, anxiety disorders, clinical depression, obsessive compulsive disorder, panic disorder, post-traumatic stress disorder, premenstrual dysphoric disorder and social phobias.

Affective disorders are known in the art to respond to treatment with antidepressants, suggesting that affective disorders share a common pathophysiology.

It should be noted herein that, as is widely accepted in the art, while the term "antidepressant" is used to describe certain compounds that have the ability to reduce depression, the same compounds may be used equally effectively against disorders not characterized by depression (e.g., anxiety), and are termed antidepressants merely because depression is perhaps the most common symptom that is treated with these same compounds.

As is further widely recognized in the art, depressive disorders represent a subfamily of affective disorders.

As used herein, the phrase "depressive disorders" describes affective disorders that are characterized by depression as a symptom.

Examples of depressive disorders include, without limitation, clinical depression, dysthymia, bipolar disorders (e.g., manic depression) and cyclothymia.

As used herein, the phrase "therapeutically effective amount" describes an amount of the compound being administered which will relieve to some extent one or more of the symptoms of the condition being treated.

As demonstrated in the examples section that follows, an exemplary therapeutically effective amount of a DLC that can be beneficially utilized in the context of the present embodiments ranges from about 0.01 mg/kg body weight to about 100 mg/kg body weight.

As used herein the term "about" refers to ± 10 %.

Further according to the present invention there is provided a use of a DLC as defined hereinbelow, in the preparation of a medicament for treating an affective disorder.

In any of the methods and uses described herein, the DLCs can be utilized either *per se* or, preferably, as a part of a pharmaceutical composition that further comprises a pharmaceutically acceptable carrier.

Thus, according to an additional aspect of the present invention, there is provided a pharmaceutical composition, which comprises one or more DLC, and a pharmaceutically acceptable carrier. The pharmaceutical composition is identified for use in the treatment of affective disorders, as defined herein.

As used herein a "pharmaceutical composition" refers to a preparation of DLCs, as described herein, with other chemical components such as pharmaceutically acceptable and suitable carriers and excipients. The purpose of a pharmaceutical composition is to facilitate administration of a compound to an organism.

Hereinafter, the term "pharmaceutically acceptable carrier" refers to a carrier or a diluent that does not cause significant irritation to an organism and does not abrogate the biological activity and properties of the administered compound. Examples, without limitations, of carriers are: propylene glycol, saline, emulsions and mixtures of organic solvents with water, as well as solid (e.g., powdered) and gaseous carriers.

Herein the term "excipient" refers to an inert substance added to a pharmaceutical composition to further facilitate administration of a compound. Examples, without limitation, of excipients include calcium carbonate, calcium phosphate, various sugars and types of starch, cellulose derivatives, gelatin, vegetable oils and polyethylene glycols.

Techniques for formulation and administration of drugs may be found in "Remington's Pharmaceutical Sciences" Mack Publishing Co., Easton, PA, latest edition, which is incorporated herein by reference.

Pharmaceutical compositions for use in accordance with the present invention thus may be formulated in conventional manner using one or more pharmaceutically acceptable carriers comprising excipients and auxiliaries, which facilitate processing of the compounds into preparations which can be used pharmaceutically. Proper formulation is dependent upon the route of administration chosen. The dosage may vary depending upon the dosage form employed and the route of administration utilized. The exact formulation, route of administration and dosage can be chosen by the individual physician in view of the patient's condition (see e.g., Fingl et al., 1975, in "The Pharmacological Basis of Therapeutics", Ch. 1 p.1).

The pharmaceutical composition may be formulated for administration in either one or more of routes depending on whether local or systemic treatment or administration is of choice, and on the area to be treated. Administration may be done orally, by inhalation, or parenterally, for example by intravenous drip or intraperitoneal, subcutaneous, intramuscular or intravenous injection, or topically (including ophtalmically, vaginally, rectally, intranasally).

Formulations for topical administration may include but are not limited to lotions, ointments, gels, creams, suppositories, drops, liquids, sprays and powders. Conventional pharmaceutical carriers, aqueous, powder or oily bases, thickeners and the like may be necessary or desirable.

Compositions for oral administration include powders or granules, suspensions or solutions in water or non-aqueous media, sachets, pills, caplets, capsules or tablets. Thickeners, diluents, flavorings, dispersing aids, emulsifiers or binders may be desirable.

Formulations for parenteral administration may include, but are not limited to, sterile solutions which may also contain buffers, diluents and other suitable additives. Slow release compositions are envisaged for treatment.

The amount of a composition to be administered will, of course, be dependent on the subject being treated, the severity of the affliction, the manner of administration, the judgment of the prescribing physician, etc.

Compositions of the present invention may, if desired, be presented in a pack or dispenser device, such as an FDA (the U.S. Food and Drug Administration) approved kit, which may contain one or more unit dosage forms containing the active ingredient. The pack may, for example, comprise metal or plastic foil, such as, but not limited to a blister pack or a pressurized container (for inhalation). The pack or dispenser device may be accompanied by instructions for administration. The pack or dispenser may also be accompanied by a notice associated with the container in a form prescribed by a governmental agency regulating the manufacture, use or sale of pharmaceuticals, which notice is reflective of approval by the agency of the form of the compositions for human or veterinary administration. Such notice, for example, may be of labeling approved by the U.S. Food and Drug Administration for prescription drugs or of an approved product insert. Compositions comprising a DLC as described herein, formulated in a compatible pharmaceutical carrier, may also be prepared, placed in an appropriate container, and labeled for treatment of an affective disorder, as is detailed herein.

Thus, according to a preferred embodiment of the present invention, the pharmaceutical composition is packaged in a packaging material and identified in print, in or on the packaging material, for use in the treatment of an affective disorder, as defined herein.

According to further embodiments, in each of the methods, uses and compositions presented herein, the DLCs can be combined with other active agents which are commonly used to treat affective disorders. These include, for example, commonly used antidepressants and anti-anxiety agents, as described hereinabove.

In any of the methods, uses and compositions described herein, the digitalis-like compound can be any compound that has a digitalis-like activity, namely, an affinity to Na⁺-K⁺-ATPase, similar to that of digitalis steroids.

The DLCs utilized in the context of the present embodiments can thus be, for example, naturally occurring DLCs, e.g., DLCs which are isolated and/or purified from an organism or a plant. Optionally and preferably, the DLCs are synthetic DLCs, designed, synthesized and demonstrated to have digitalis-like activity. In any event, the methods, uses and compositions described herein utilize exogenous DLCs.

Exemplary DLCs which can be utilized in the context of the preset embodiments include, but are not limited to, isolated cardenolides and bufadienolides, and synthetic DLCs, being preferably derivatives of 19-norbufalin.

Of the presently known and practiced DLCs, substances such as ouabain, digoxin, bufalin and marinobufogenin can be utilized in the context of the present embodiments.

Other compounds which have been proposed to act as DLCs and can be utilized in the context of the present embodiments include unsaturated fatty acids (such as described, for example, in Bidard, J. N., et al. (1984) Biochim. Biphys. Acta 769: 245-252; Tamura, M., et al. (1985) J. Biol. Chem. 260: 9672-9677; and Kelly, R. A., et al. (1986) J. Biol. Chem. 261: 11704-11711), hydroxy unsaturated fatty acids (such as described, for example, in Lichtstein, D., et al. (1991) J. Endocrinol. 128: 71-78), lysophosphatidylcholines (such as described, for example, in Tamura, M., et al. (1987) Biochemistry 26: 2797-2806), dopamine [see, Clarkson, E. M. & De Wardner, H. E. (1985) Clinical and Experimental Hypertension Part A, A7, 673-683], dehydroepiandrosterone sulfate [see, Vasdev, S., et al. (1985) Res. Commun. Chem. Path. and Pharmacol. 49: 387-399], lignan [see, Fagoo, M., et al. (1986) Biochem. Biophys. Res. Commun. 134: 1064-1070] and ascorbic acid [see, Ng, Y. C., et al. (1985) Biochem. Pharmacol 34: 2525-2530]. Additional, commercially available DLCs include digitalis glycosides such as Lanoxin® and Lanoxicaps®.

In a preferred embodiment of the present invention, the digitalis-like compound is a 19-norbufalin derivative, preferably a 19-norbufalin derivative such as described in U.S. Patent No. 7,087,590.

Preferred DLCs according to the present embodiments can be collectively represented by the general Formula I: 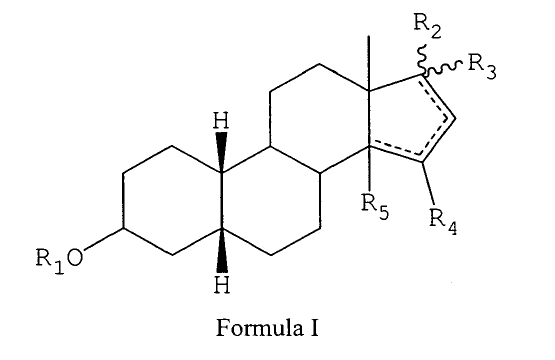 wherein:
R₁ is, for example, hydrogen, alkyl, alkenyl, cycloalkyl or a hydroxy protecting group, or, alternatively, is absent; when R₁ is absent, the oxygen is linked to the steroidal ring via a double bond, forming an oxo group;
R₂ is, for example, hydrogen, hydroxy, thiol, alkoxy, thioalkoxy, aminoalkyl or absent;
R₃ is selected from the group consisting of furyl, dihydrofuryl, tetrahydrofuryl, pyranyl, dihydropyranyl, tetrahydropyranyl, pyridanyzil, lactone and the like, optionally being substituted;
R₄ is hydrogen or hydroxy, or, alternatively, forms a 3-membered ring with R₅; and
R₅ is hydrogen, hydroxy or absent, or, alternatively, forms a 3-membered ring with R₅.

The dashed line in Formula I represents an optional double bond.

Each of the curved lines represents a substituent located above the plane of the steroid (a bond represented by 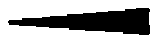 ), or below the plane (a bond represented by 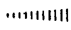 ).

The numbers 14, 15 and 16 in general Formula I above mark the carbon atoms of positions 14, 15 and 16, respectively.

Positions in general Formula I that are not marked as substituted by particular substituents (R₁-R₅) preferably bear hydrogen atoms, but can optionally be substituted by other substituents such as, for example, halo, alkyl, alkoxy, alkenyl, cyano, nitro, haloalkyl, hydroxy, thiol, thioalkoxy and others, as long as these substituents do not affect, or interfere with, the desired biological effect of the compound.

It is noted that the feasibility for some of the substituents to be located at the indicated positions depends on the valence of the substituted position. For example, R₂, R₃ and R₅ may be absent if the position to which they are substituted contains a double bond.

The 3-membered ring formed between R₄ and R₅ can be a cyclopropyl ring, or optionally and preferably, an oxirane ring.

The phrase "hydroxy protecting group", as used herein, refers to a substituent of a hydroxy group that is employed to protect or block a hydroxy group and which can be removed, preferably under mild conditions, to re-generate a hydroxy group, if desired. The choice of a suitable hydroxy protecting group is within the knowledge of one of ordinary skill in the art.

Exemplary hydroxy protecting groups that are suitable for use in the context of the present embodiments include, but are not limited to, alkyl esters, aryl esters, alkyl silanes, aryl silanes, alkylaryl silanes, alkyl carbonates, aryl carbonates, benzyl, substituted benzyl, ethers, and substituted ethers.

These groups can optionally by substituted by e.g., halo, alkyl, haloalkyl, alkoxy, cycloalkyl, nitro, cyano, aryl, aryloxy and the like. For a general description of protecting groups and their use, see, for example, T. W. Greene, Protective Groups in Organic Synthesis, John Wiley & Sons, New York, 1991.

Additional hydroxy protecting groups that are suitable for use in the context of the present invention include amino acids, peptides and saccharides, and any other groups that can be removed under mild, preferably physiological, conditions.

Preferred hydroxy protecting groups according to the present embodiments include, but are not limited to, benzyl, amino acids, peptides, preferably comprising from 2 to 20 amino acid residues, and mono- and di-saccharides.

The term "hydroxy" or "hydroxyl", as used herein, refers to an -OH group.

The term "thiohydroxy" or "thiol", as used herein, refers to a -SH group.

The term "benzyl", as used herein, refers to -CH₂C₆H₅. A benzyl group can be unsubstituted or substituted with one or more suitable substituents.

As used herein, the terms "halo" and "halide", which are referred to herein interchangeably, describe an atom of a fluorine, chlorine, bromine or iodine, also referred to herein as fluoride, chloride, bromide and iodide.

As used herein, the term "alkyl" describes an aliphatic hydrocarbon including straight chain and branched chain groups. Preferably, the alkyl group has 1 to 10 carbon atoms, and more preferably 1-6 carbon atoms. Whenever a numerical range; e.g., "1-10", is stated herein, it implies that the group, in this case the alkyl group, may contain 1 carbon atom, 2 carbon atoms, 3 carbon atoms, etc., up to and including 10 carbon atoms. The alkyl can be substituted or unsubstituted. When substituted, for example, by halo, hydroxy or amine, the alkyl is referred to herein as haloalkyl, hydroxyalkyl or aminoalkyl, respectively. Other substituents can be selected from, for example, alkoxy, alkenyl, cyano, nitro, haloalkyl, thiol, thioalkoxy, esters, aryls and others.

The term "alkenyl" describes an alkyl having at least two carbon atoms and at least one carbon-carbon double bond.

The term "cycloalkyl" describes an all-carbon monocyclic or fused ring (i.e., rings which share an adjacent pair of carbon atoms) group where one or more of the rings does not have a completely conjugated pi-electron system. The cycloalkyl group may be substituted or unsubstituted.

The term "aryl" describes an all-carbon monocyclic or fused-ring polycyclic (i.e., rings which share adjacent pairs of carbon atoms) groups having a completely conjugated pi-electron system. The aryl group may be substituted or unsubstituted.

The term "halide" and "halo" describes fluorine, chlorine, bromine or iodine atom.

The term "haloalkyl" describes an alkyl group as defined above, further substituted by one or more halides.

The term "carbonyl" or "carbonate" as used herein, describes a -C(=O)-R' group, with R' being hydrogen, alkyl, cycloalkyl or aryl, as defined herein.

The term "oxo" describes a =O group.

The term "oxirane" describes a 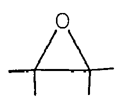 group.

The term "alkoxy" describes both an -O-alkyl and an -O-cycloalkyl group, as defined herein.

The term "thioalkoxy" describes both an -S-alkyl and an -S-cycloalkyl group, as defined herein.

The term "aryloxy" describes both an -O-aryl and an -O-heteroaryl group, as defined herein.

The term "cyano" describes a -C=N group. The term "isocyanate" describes an -N=C=O group. The term "nitro" describes an -NO₂ group.

The term "acyl halide" describes a -(C=O)-X group wherein X is halide, as defined hereinabove.

The term "ester" describes a -C(=O)-OR' group or a -OC(=O)R', where R' is as defined herein.

The term "silyl" or "silane" describes a -SiR'₃ group, whereby R' is as defined herein.

The phrase "amino acid", as used herein, includes the 20 naturally occurring amino acids; those amino acids often modified post-translationally *in vivo*, including, for example, hydroxyproline, phosphoserine and phosphothreonine; and other unusual amino acids including, but not limited to, 2-aminoadipic acid, hydroxylysine, isodesmosine, nor-valine, nor-leucine and ornithine. Furthermore, the term "amino acid" includes both D- and L-amino acids.

The term "peptide", as used herein, encompasses any sequence of two or more amino acids, as defined herein, linked therebetween via a peptide bond or a modification thereof, and include native peptides (either degradation products, synthetically synthesized peptides or recombinant peptides) and peptidomimetics (typically, synthetically synthesized peptides). The peptide can be a linear peptide or a cyclic peptide. Preferably, the peptide is a short peptide, having from 2 to 20 amino acid residues.

The term "saccharide", as used herein, describes compounds composed of one or more saccharide units, and is preferably a monosaccharide or a disaccharide. As is known in the art, monosaccharides consist of a single saccharide molecule which cannot be further decomposed by hydrolysis. Representative examples of monosaccharides include, without limitation, pentoses such as, but limited to, arabinose, xylose, and ribose. Representative examples of disaccharides include, but not limited to, sucrose, maltose, lactose, and cellobiose.

The term "furyl", as used herein, describes a 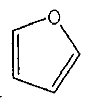 group. The furyl can be linked to the steroidal skeleton via each of the carbon atoms and is preferably linked to the steroidal skeleton via the carbon at position 3 thereof. The furyl can be substituted or non-substituted. When substituted, the substituent can be, for example, alkyl, hydroxy, hydroxyalkyl, alkoxy, carbonyl, ester, dioxolane, halo, haloalkyl, thiol, and the like.

The terms "dihydrofuryl" and "tetrahydrofuryl", as used herein, describe a partially saturated (having a single double bond) or a completely saturated furyl, as defined herein, optionally being substituted by, for example, alkyl, hydroxy, hydroxyalkyl, alkoxy, carbonyl, ester, dioxolane, halo, haloalkyl, thiol, and the like.

The term "pyranyl" describes a 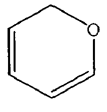 group. The pyranyl can be linked to the steroidal skeleton via each of the carbon atoms and is preferably linked to the steroidal skeleton via the carbon at position 3 thereof. The pyranyl can be substituted or non-substituted. When substituted, the substituent can be, for example, alkyl, hydroxy, hydroxyalkyl, alkoxy, carbonyl, ester, dioxolane, halo, haloalkyl, thiol, and the like.

The terms "dihydropyranyl" and "tetrahydropyranyl", as used herein, describe a partially saturated (having a single double bond) or a completely saturated pyranyl, as defined herein, optionally being substituted by, for example, alkyl, hydroxy, hydroxyalkyl, alkoxy, carbonyl, ester, dioxolane, halo, haloalkyl, thiol, and the like.

The term "lactone", as used herein, describes a saturated or non-saturated furyl or pyranyl, as defined herein, substituted by an oxo group, as defined herein.

In preferred embodiments of the present invention, the DLCs are 19-norbufalin derivatives having general Formula I above, in which:
R₁ is hydrogen or a hydroxy protecting group;
R₂ is hydrogen, hydroxy or absent;
R₃ is selected from the group consisting of: 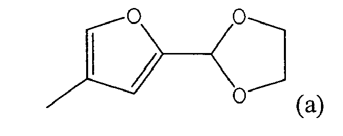 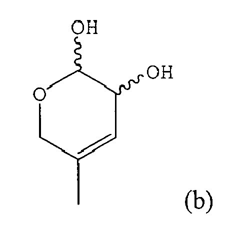 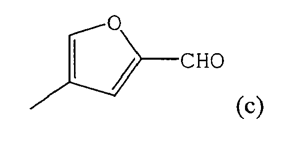 and 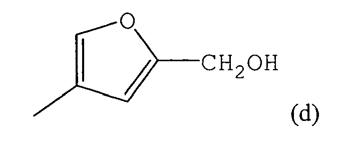
R₄ is hydrogen or hydroxy; and
R₅ is hydrogen, hydroxy or absent,
whereas the dashed line in Formula I represents an optional double bond.

Alternatively, compounds having general Formula I hereinabove are androstane derivatives such as those described in U.S. Patents Nos. 5,5567,679 and 5,591,734, which are incorporated by reference as if fully set forth herein.

The compounds represented by Formula I hereinabove can be in any isomeric form thereof and hence can be, for example, in a form of an α-isomer (alpha isomer) or a β-isomer (beta isomer).

According to preferred embodiments of the present invention, the compound of Formula I is a 19-norbufalin derivative, as described herein, being in a form of an α-isomer thereof.

As used herein, the terms "α-isomer" and "β-isomer" refer to the configuration of bond of the -OR₁ group at position 3 (namely, 3α- and 3β- isomers). As commonly used in the nomenclature of steroids, β refers to a substituent located above the plane of the steroid, (typically, bonds represented by 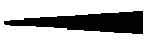 ), while α refers to a substituent below the plane (typically, bonds represented by 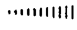 ). It is noted that such nomenclature is only meaningful when presenting the molecular structure while using the agreed, standard orientation for steroid molecules, as in Formula I herein, in order to define which side of the plane is "above" and which side is "below".

Without being bound to any particular theory, it is suggested that natural, endogenous DLCs, which are known to be β-isomers, may promote affective disorders or at least be present in elevated levels in brains of subjects suffering an affective disorder. α-Isomers of DLCs such as the 19-norbyfalin derivatives described herein are believed to bind the same receptors as do β-isomers, but to affect different pathways, and thus to be devoid of the activity of β-isomers. α-Isomers of DLCs such as the 19-norbyfalin derivatives described herein are further believed to antagonize the activity of endogenous DLCs and may thereby serve as antagonists against the mechanism behind affective disorders.

According to preferred embodiments of the present invention, in compounds having general Formula I above, R₁ is benzyl. Preferably, in such compounds, R₂ is OH.

In such compounds, R₃ is preferably 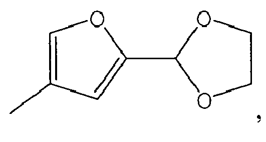 referred to herein as group (a) or simply as (a).

Further preferably, in such compounds, R₄ and R₅ are each hydrogen and the compound has a double bound between the carbons at the 15 and 16 positions.

Alternatively, in such compounds, R₄ is hydrogen and R₅ is absent, and the compound has a double bond between carbon atoms at the 14 and 15 positions.

Further alternatively, in such compounds, R₄ is hydroxy, and R₅ is hydrogen and the compound is saturated.

According to preferred embodiments of the present invention, in compounds having general Formula I above, R₁ is benzyl, R₂ is OH, and R₃ is preferably 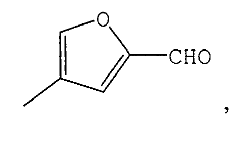 also referred to herein as group (c) or simply as (c).

In such compounds, preferably, R₄ is hydrogen and R₅ is absent, and the compound has a double bond between the carbon atoms at the 14 and 15 positions.

Further according to preferred embodiments, R₁ is hydrogen and preferably, R₂ is hydrogen.

In such compounds R₃ is preferably 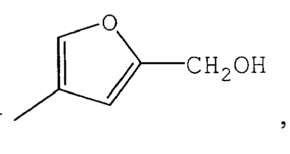 also referred to herein as group (d) or simply as (d)..

In such compounds, preferably, R₄ is hydroxy and R₅ is hydrogen and the compound is saturated.

Further according to preferred embodiments, R₁ is hydrogen , R₂ is hydrogen and R₃ is preferably 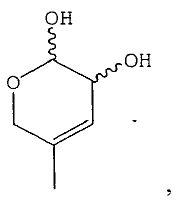 also referred to herein as group (b), or simply as (b).

In such compounds, preferably, R₄ is hydrogen and R₅ is absent, and the compound has a double bond between the carbon atoms at the 14 and 15 positions.

Further according to preferred embodiments, R₁ is hydrogen , R₂ is hydrogen and R₃ is preferably (c).

In such compounds, preferably, R₄ is hydrogen and R₅ is hydroxy.

According to a particular preferred embodiment of the present invention, the digitalis-like compound has general Formula I above, and has benzyl for R₁, OH for R₂, (a) for R₃, hydrogen for both R₄ and R₅, and a double bond between the carbons at the 15 and 16 positions. This compound is also referred to herein as Compound 13-3.

According to another preferred embodiment of the present invention, the digitalis-like compound has general Formula I above, and has benzyl for R₁, hydrogen for R₂, OH for R₄ and hydrogen for R₅.

According to yet another preferred embodiment of the present invention, the digitalis-like compound has general Formula I above, and has hydrogen for R₁, hydrogen for R₂, (d) for R₃, OH for R₄ and hydrogen for R₅.

According to still yet another preferred embodiment of the present invention, digitalis-like compound has general Formula I above, and has benzyl for R₁, hydrogen for R₂, (b) for R₃, hydrogen for R₄ and a double bond between the carbons at the 14 and 15 positions.

According to still yet another preferred embodiment of the present invention, digitalis-like compound has general Formula I above, and has benzyl for R₁, hydrogen for R₂, (c) for R₃, hydrogen for R₄ and a double bond between the carbons at the 14 and 15 positions.

According to a further preferred embodiment of the present invention, the digitalis-like compound has general Formula I above, and has hydrogen for R₁, hydrogen for R₂, (c) for R₃, hydrogen for R₄ and OH for R₅.

The 19-norbufalin derivatives described herein, as well as the preparation thereof, are described in detail in U.S. Patent No. 7,087,590.

The DLCs utilized in the various embodiments of the present invention, as described herein, can further be in a form of prodrugs, hydrates, solvates or pharmaceutically acceptable salts thereof, as defined herein.

The term "prodrug" refers to an agent, which is converted into the active compound (the active parent drug) *in vivo*. Prodrugs are typically useful for facilitating the administration of the parent drug. They may, for instance, be bioavailable by oral administration whereas the parent drug is not. A prodrug may also have improved solubility as compared with the parent drug in pharmaceutical compositions. Prodrugs are also often used to achieve a sustained release of the active compound *in vivo.* An example, without limitation, of a prodrug would be a compound as described herein, having one or more carboxylic acid or hydroxyl moieties, which is administered as an ester (the "prodrug"). Such a prodrug is hydrolyzed *in vivo*, to thereby provide the free compound (the parent drug). The selected ester may affect both the solubility characteristics and the hydrolysis rate of the prodrug.

The term "solvate" refers to a complex of variable stoichiometry (e.g., di-, tri-, tetra-, penta-, hexa-, and so on), which is formed by a solute (the DLC described herein) and a solvent, whereby the solvent does not interfere with the biological activity of the solute. Suitable solvents include, for example, ethanol, acetic acid and the like.

The term "hydrate" refers to a solvate, as defined hereinabove, where the solvent is water.

As used herein, the phrase "pharmaceutically acceptable salt" refers to a charged species of the parent compound and its counter-ion, which is typically used to modify the solubility characteristics of the parent compound and/or to reduce any significant irritation to an organism by the parent compound, while not abrogating the biological activity and properties of the administered compound.

The DLCs utilized in the various embodiments of the present invention, as described herein, can further be in a form of any tautomeric, enantiomeric and/or diasteromeric isomers thereof.

Additional objects, advantages, and novel features of the present invention will become apparent to one ordinarily skilled in the art upon examination of the following examples, which are not intended to be limiting. Additionally, each of the various embodiments and aspects of the present invention as delineated hereinabove and as claimed in the claims section below finds experimental support in the following examples.

### EXAMPLES

Reference is now made to the following examples, which together with the above descriptions, illustrate the invention in a non limiting fashion.

### MATERIALS AND EXPERIMENTAL METHODS

### Compounds:

4-(3'β,15'β-dihydroxy-5β-estran-17β-yl)furan-2-methyl alcohol (Compound 13-3-03, an α isomer of Compound 13-3, was prepared as described in U.S. Patent No. 7,087,590 or in Deutsch et al., J. Med. Chem. 49:600-606 (2006).

### Animals:

Sprague-Dawley rats weighing 100 to 200 grams were housed according to a 12-hour light/dark cycle and allowed a 5-day acclimatization period with normal rat chow and tap water. All procedures were carried out according to the guidelines of the Hebrew University-Hadassah Medical School Animal Care Committee for the use and care of laboratory animals.

### Forced-swimming rat model:

The forced-swimming test (FST) is an animal model widely used to evaluate the efficacy of antidepressant treatments [see, for example, Porsolt et al. Nature 1977, 266:730-732]. In this behavioral paradigm a passive behavior, immobility, is considered to reflect behavioral despair [Porsolt et al. Eur JPharmacol 1979, 57:201-210; Porsolt and Len'egre in: Experimental Approaches to Anxiety and Depression 1992, John Wiley & Sons Ltd., pp 73-85]. When antidepressant drugs are administered, a reduction in immobility is produced and is thought to reflect an antidepressant-like action. Non-pharmacological antidepressant treatments, such as sleep deprivation and electroconvulsive shock also reduce immobility behavior in this model [Porsolt et al. Nature 1977, 266:730-732]. In addition, FST proved to be sensitive to physiological changes such as variation in steroid levels along the oestrous cycle [Contreras et al. Biol Psychiatry 1998, 22:1121-1128; Contreras et al. Physiol Behav 2000, 68:279-284]. Based on these characteristics, the forced swimming test was selected as a model to measure depressive-like behavior in this study

Thus, the traditional forced swim test was performed as originally developed by Porsolt et al. (1977), with minor modifications. The test was conducted in a cylinder plastic swim tank (20 cm diameter × 40 cm high) with no top. The tank was filled with 27 °C (± 2 °C) water to a depth of 30 cm. Rats were placed into a swim tank for 15 minutes on day 1 to induce a state of "helplessness". The rats were then dried off with a towel, and placed back into their home cage to dry under a heat lamp for 20 minutes. On day 2, Compound **13-3-03** dissolved in a solution containing 1 % pluronic acid and 2 % ethanol at a concentration of 100µM or the solvent only were injected intraperitoneally (1 ml). The injections were done under light ether anesthesia. Two hours after the injections, the rats were returned to the swim tank for 5 minutes, during which the rat's behavior was recorded with a video camera. At the end of the 5 minutes period, the rat was removed from the tank and euthanized by cervical dislocation. The water in the tank was changed after each rat. Behaviors scored in the test included: (1) time spent climbing: observed when the rat's forepaws break the surface of the water and the rat actively struggles/searches to get out of the tank; (2) time spent swimming: observed when the rat makes active swimming motions that are beyond those needed to simply stay afloat but less than those observed with struggling; and (3) time spent immobile: observed when the rat makes very little (but enough to keep from drowning) movement with its body. The time to the first period (10 seconds) of immobility (TFIP) was scored as well.

### EXPERIMENTAL RESULTS

The data obtained in the force swimming test is presented in Figure 1.

As shown in Figure 1, the immobility period which is correlated to depression was significantly lower with the tested, strongly indicating it has an anti-depressive effect. Thus, Immobility period, following administration of Compound 13-3 was significantly lower than control *p<0.000326 and climbing period, mobility and TFIP (time to the first period 10 seconds of immobility) were significantly higher than control, **p<0.0094, *p<0.000326, ***p<0.017, respectively.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination.

Although the invention has been described in conjunction with specific embodiments thereof, it is evident that many alternatives, modifications and variations will be apparent to those skilled in the art. Accordingly, it is intended to embrace all such alternatives, modifications and variations that fall within the spirit and broad scope of the appended claims.

All publications, patents and patent applications mentioned in this specification are herein incorporated in their entirety by reference into the specification, to the same extent as if each individual publication, patent or patent application was specifically and individually indicated to be incorporated herein by reference. In addition, citation or identification of any reference in this application shall not be construed as an admission that such reference is available as prior art to the present invention.

## Claims

1. Use of a digitalis-like compound having the general Formula I: 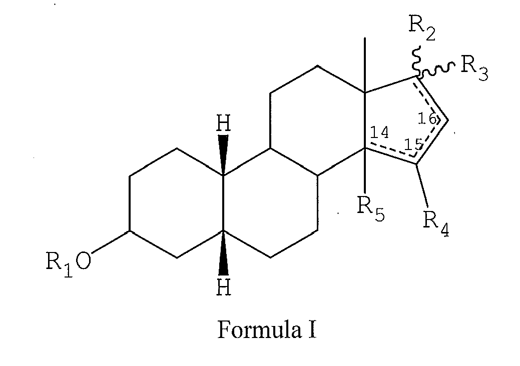 wherein:
the dashed line represents an optional double bond;
R₁ is hydrogen, alkyl, alkenyl, cycloalkyl or a hydroxy protecting group or is absent;
R₂ is hydrogen, hydroxy, alkoxy, aminoalkyl or absent;
R₃ is selected from the group consisting of furyl, dihydrofuryl, tetrahydrofuryl, pyranyl, dihydropyranyl, tetrahydropyranyl, pyridanyzil and lactone;
R₄ is hydrogen or hydroxy, or, alternatively, forms a 3-membered ring with R₅; and
R₅ is hydrogen, hydroxy or absent, or, alternatively, forms a 3-membered ring with R₄,
in the manufacture of a medicament for treating an affective disorder.

2. The use of claim 1, wherein said affective disorder is a depressive disorder.

3. The use of claim 1, wherein said affective disorder is selected from the group consisting of a body dysmorphic disorder, a bulimia nervosa, an eating disorder, cataplexy, a general anxiety disorder, a depressive disorder, an obsessive compulsive disorder, a panic disorder, a post-traumatic stress disorder, a premenstrual dysphoric disorder, and a social phobia.

4. The use of any of claims 2 and 3, wherein said depressive disorder is selected from the group consisting of clinical depression, dysthymia and a major depressive disorder.

5. The use of any of claims 1-4, wherein:
R₁ is hydrogen or a hydroxy protecting group;
R₂ is hydrogen, hydroxy or absent;
R₃ is selected from the group consisting of: 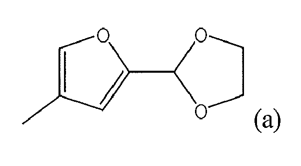 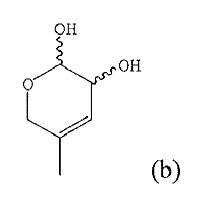 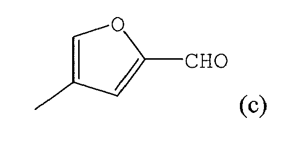 and 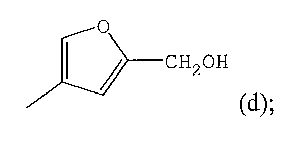
R₄ is hydrogen or hydroxy; and
R₅ is hydrogen, hydroxy or absent.

6. The use of claim 5, wherein said compound is in a form of an alpha isomer or a beta isomer thereof.

7. The use of claim 6, wherein said compound is in a form of an alpha-isomer thereof.

8. The use of claim 5, wherein said hydroxy protecting group is selected from the group consisting of benzyl, amino acid, peptide, and mono- and di-saccharide.

9. The use of claim 8, wherein R₁ is benzyl.

10. The use of claim 9, wherein R₂ is OH.

11. The use of claim 5, wherein R₁ is hydrogen.

12. The use of claim 11, wherein R₂ is hydrogen.

13. The use of claim 11, wherein R₃ is (d).

14. The use of claim 10, wherein R₃ is selected from the group consisting of (a), (b) and (c).

15. The use of claim 12, wherein R₃ is (c).
